# EUROPEAN PATENT APPLICATION

(11) **EP 2 425 808 A1**
(43) Date of publication of application: **07.03.2012**
(21) Application number: 11006992.9
(22) Date of filing: 26.08.2011
(51) Int. Cl.: A61K 6/083

(54) **Polymerizable composition**

(30) Priority: 03.09.2010 JP 2010197362
(71) Applicant: GC Corporation, Bunkyo-ku Tokyo 113-0033 (JP)
(72) Inventor: Tokui, Hideki, Tokyo (JP)
(74) Representative: Müller-Boré & Partner Patentanwälte

(57) **Abstract**

To provide a polymerizable composition excellent in curability under a humid condition such as in an oral cavity, the polymerizable composition includes a first component and a second component, the first component includes cumene hydroperoxide as a peroxide in (meth)acrylate, the second component includes a thiourea derivative as a reductant and a vanadium compound as a polymerization accelerator in (meth)acrylate and the vanadium compound is preferably one or more kinds selected from vanadium acetylacetonate, vanadyl acetylacetonate, vanadyl stearate, vanadium naphthenate, and vanadium benzoyl acetonate.

## Description

The present invention relates to a polymerizable composition to mix two or more kinds of liquids or pastes and polymerize them. More particularly, the present invention relates to a dental polymerizable composition being the most suitable for uses which need high curability under a humid condition such as in an oral cavity at a time of a dental treatment.

In a dental treatment field, prostheses such as a crown, a bridge, an inlay, and the like have been used to restore or replace a lost tooth or oral tissues. As a material for bonding these prostheses to a tooth, a composition including a polymerizable resin, a filler particle, and the like has been mainly used, and such the composition is cured by a mechanism of ordinary temperature (chemical) polymerization, photopolymerization, or the like.

As a method for polymerizing a polymerizable composition including a monomer, an oligomer, and a prepolymer of methacrylate, acrylate, or the like which have a radical polymerization property at ordinary temperature, a method combining to use an organic peroxide and aromatic tert-amine, as a chemical polymerization catalyst, has been conventionally used (e.g., refer to Japanese Patent Application Laid-Open No. H10-338610). In this method, there is no problem in polymerizability of monomers under the condition of no water. However, under the humid condition such as in the oral cavity, there is a problem that polymerization of the monomers is often prevented.

A polymerizable composition containing a ternary catalyst including a pyrimidinetrione derivative, an organohalogen compound, and a copper ionic compound or an iron ionic compound has been disclosed (e.g., refer to Japanese Patent Application Laid-Open No. H11-228330). However, this composition is not enough in polymerizability under the condition of coexistance of water.

Furthermore, polymerization initiator system combining tert-butyl hydroperoxide, a thiourea derivative, and a vanadium compound has been disclosed (e.g., refer to Japanese Patent Application Laid-Open No. 2009-144054). A composition using this polymerization initiator system has proper storing stability and excellent curability. However, the polymerization of the monomers tends to be prevented under the condition where water content exists such as in the oral cavity, too.

An objective of the present invention is to provide an excellent polymerizable composition in which polymerization is not prevented even under a humid condition such as in an oral cavity.

Present inventors carried out earnest works to solve the aforementioned problems and, as a result, they found out the following to complete the present invention. A polymerizable composition in which polymerization is not prevented even under the humid condition such as in the oral cavity can be obtained by using cumene hydroperoxide as a peroxide, a thiourea derivative as a reductant, and a vanadium compound as a polymerization accelerator.

More specifically, the present invention is a polymerizable composition including a first component and a second component, where the first component includes cumene hydroperoxide as a peroxide in (meth)acrylate, and the second component includes a thiourea derivative as a reductant and a vanadium compound as a polymerization accelerator in (meth)acrylate.

The polymerizable composition according to the present invention is a polymerizable composition in which polymerization is not prevented even under the humid condition as in the oral cavity.

Preferred embodiments of the present invention will be described in detail below.

(Meth)acrylate in the present invention means various kinds of monomers, oligomers and prepolymers of acrylate or methacrylate. More particularly, (meth)acrylate used in the present invention could be methyl (meth)acrylate, ethyl (meth)acrylate, isopropyl (meth)acrylate, n-butyl (meth)acrylate, isobutyl (meth)acrylate, hydroxypropyl (meth)acrylate, tetrahydrofurfuryl (meth)acrylate, glycidyl (meth)acrylate, 2-hydroxyethyl (meth)acrylate, 2-hydroxypropyl (meth)acrylate, 3-hydroxypropyl (meth)acrylate, 2-methoxyethyl (meth)acrylate, 2-ethoxyethyl (meth)acrylate, 2-ethylhexyl (meth)acrylate, benzyl (meth)acrylate, 2-hydroxy-1,3-di(meth)acryloxy propane, ethylene glycol di(meth)acrylate, diethylene glycol di(meth)acrylate, triethylene glycol di (meth) acrylate, butylene glycol di(meth)acrylate, neopentyl glycol di(meth)acrylate, 1, 3-butanediol di(meth)acrylate, 1, 4-butanediol di(meth)acrylate, 1, 6-hexanediol di(meth)acrylate, trimethylolpropane tri(meth)acrylate, trimethylolethane tri(meth)acrylate, pentaerythritol tri(meth)acrylate, trimethylolmethane tri(meth)acrylate, pentaerythritol tetra(meth)acrylate, polybutylene glycol di(meth)acrylate, or bisphenol A glycidyl (meth)acrylate. Monomers, oligomers, and prepolymers of these compounds can be properly used. Further, as for (meth)acrylate having urethane bond, di-2-(meth)acryloxyethyl-2, 2, 4-trimethylhexamethylene dicarbamate, 1, 3, 5-tris [1, 3-bis{(meth)acryloyloxy}-2-propoxycarbonylaminohexan e]-1, 3, 5-(1H, 3H, 5H) triazine-2, 4, 6- trione, and 2, 2-bis-4-(3-(meth)acryloxy-2-hydroxypropyl)-phenyl propane, and the like can be used. In addition, the (meth)acrylate having urethane bond could be (meth)acrylate of urethane oligomer including 2, 2'-di(4-hydroxycyclohexyl) propane, 2-oxypanone, hexamethylene diisocyanate, and 2-hydroxyethyl (meth)acrylate, and (meth)acrylate of urethane oligomer including 1, 3-butanediol, hexamethylene diisocyanate, and 2-hydroxyethyl (meth)acrylate. These (meth)acrylates and acrylates can be used independently or by mixing two or more kinds.

In the present invention, (meth)acrylate having an acid group can be used. Cumene hydroperoxide is stable to both (meth) acrylate not having an acid group and (meth)acrylate having an acid group. (Meth)acrylate having an acid group has effect for giving an adhesive property to the polymerizable composition to adhere to a tooth, dental restorative materials which are ceramics such as zirconia or alumina, and an alloy including noble metals. (Meth)acrylate having an acid group is preferably (meth) acrylate having a phosphate group or a carboxyl group. Thus, (meth)acrylate having one or plural phosphate groups or carboxyl groups in one molecule can be used. Since the phosphate group has acidity stronger than the carboxyl group, the phosphate group has higher effect for dissolving a smear layer of a tooth surface and for tooth demineralization. Particularly, the phosphate group can exercise an effect for highly improving adhesive property to enamel. (Meth)acrylate having a phosphate group could be 2-(meth)acryloyloxyethyldihydrogen phosphate, bis[2-(meth)acryloyloxyethyl]hydrogen phosphate, 2-(meth)acryloyloxyethylphenylhydrogen phosphate, 6-(meth)acryloyloxyhexyldihydrogen phosphate, 6-(meth)acryloyloxyhexylphenylhydrogen phosphate, 10-(meth)acryloyloxydecyldihydrogen phosphate, 1,3-di(meth)acryloylpropane-2-dihydrogen phosphate, 1, 3-di(meth)acryloylpropane-2-phenylhydrogen phosphate, bis[5-{2-(meth)acryloyloxyethoxycarbonyl} heptyl] hydrogen phosphate, (2-propenoic acid, 2-methyl)-phosphinicobis(oxy-2,1,3-propane), 2-(phosphonoxy)-1,3-propanediyl bismethacrylate or the like. Particularly, 10-(meth)acryloyloxydecyldihydrogen phosphate is preferable because of having an excellent adhesive property and stability of the (meth) acrylate itself. The (meth) acrylate having the phosphate group can be used alone or by mixing two or more kinds.

(Meth)acrylate having the carboxyl group could be 4-(meth)acryloxyethyltrimellitic acid, 4-(meth)acryloxyethyltrimellitic acid anhydride, 4-(meth)acryloxydecyltrimellitic acid, 4-(meth)acryloxydecyltrimellitic acid anhydride, 11-(meth)acryloyloxy-1,1-undecanedicarboxylic acid, 1, 4-di(meth)acryloyloxypyromellitic acid, 2-(meth)acryloyloxyethylmaleic acid, 2-(meth)acryloyloxyethylphthalic acid, 2-(meth)acryloyloxyethylhexahydrophthalic acid, or the like. Particularly, 4-(meth)acryloxyethyltrimellitic acid and 4 -(meth)acryloxyethyltrimellitic acid anhydride are preferable in that these have an excellent adhesive property.

In the polymerizable composition according to the present invention, cumene hydroperoxide as a peroxide is blended with (meth) acrylate in the first component, a thiourea derivative as a reductant, and a vanadium compound as a polymerization accelerator are blended in the second component, and oxidation/reduction reactions of cumene hydroperoxide as a peroxide and a thiourea derivative as a reductant are utilized.

Cumene hydroperoxide blended with the polymerizable composition according to the present invention is a peroxide. The blending amount of cumene hydroperoxide is preferably 0.1 to 10% by weight in the first component. If the blending amount is less than 0.1% by weight, a function as a peroxide tends to be insufficient. If the blending amount exceeds 10% by weight, (meth)acrylate in the first component is polymerized easily, so that storing stability of the composition tends to decrease.

The thiourea derivative used in the polymerizable composition according to the present invention is a reductant, and is stable in (meth)acrylate. The content of the thiourea derivative in the second component is preferably 0.1 to 10% by weight. If the content is less than 0.1% by weight, the ability as a reductant is insufficient. If the content exceeds 10% by weight, the thiourea derivative could not be dissolved in (meth) acrylate. The thiourea derivatives could be ethylenethiourea, diethylthiourea, tetramethylthiourea, N-acetylthiourea, N-benzoylthiourea, diphenylthiourea, dicyclohexylthiourea, or the like. Particularly, N-acetylthiourea and N-benzoylthiourea are preferable.

The vanadium compound blended in the polymerizable composition according to the present invention is a polymerization accelerator, and is stable in (meth)acrylate. The blending amount of the vanadium compound in the second component is preferably 0.001 to 1% by weight. If the blending amount is less than 0.001% by weight, the effect as the polymerization accelerator tends to be insufficient. If the blending amount exceeds 1% by weight, the second component looks to be colored in dark green, or the (meth) acrylate may be polymerized while the component is stored. The vanadium compound could be vanadium acetylacetonate, vanadyl acetylacetonate, vanadyl stearate, vanadium naphthenate, vanadium benzoyl acetonate, or the like. Particularly, vanadium acetylacetonate and vanadyl acetylacetonate are preferable.

In the polymerizable composition according to the present invention, a filler can be added to one or both of the first component and the second component to increase the strength. For example, when the polymerizable composition is used as a dental adhesive composition, a filler is blended to make each component to be in a paste state, so that operability can be increased. The filler could be powder of anhydrous silicic acid, glasses such as barium glass, alumina glass,potassium glass,fluoroaluminosilicateglass, and the like, synthetic zeolite, calcium phosphate, feldspar, fumed silica, aluminum silicate, calcium silicate, magnesium carbonate, hydrous silicic acid, hydrous calcium silicate, hydrous aluminum silicate, quartz, or the like. In order to bond with (meth)acrylate, the filler can be subjected to a surface treatment with a silane coupling agent, such as γ-methacryloxypropyltrimethoxysilane, vinyltrichlorosilane, vinyltriethoxysilane, vinyltrimethoxysilane, vinyltriacetoxysilane or the like. Further, an organic and inorganic composite filler produced by previously mixing the aforementioned filler with monomers and oligomers, curing the mixture, and pulverizing the cured body, can be used. These fillers can be used independently or by mixing two or more. Particularly, anhydrous silicic acid, fumed silica and quartz are the most stable when coexisting with an acid component. In addition, of course, different fillers can be used respectively in each component of the first component and the second component which are used in the present invention.

The mixing ratio of the first component and the second component in the polymerizable composition according to the present invention is preferably 10:1 to 1:10 by weight. If the ratio is out of this range, the balance of the polymerization catalyst in each component comes to be hardly kept, so that a problem in polymerization could occur. Mixing of the polymerizable composition according to the present invention can be done by an operator manually using a spatula and a kneading paper, or using an auto mixing system with a mixing tip.

The polymerizable composition according to the present invention can, of course, properly include a photopolymerization catalyst, an antibacterial agent, a pigment and the like, which are conventionally used, if necessary. Further, in order to increase reactivity with respect to tooth of (meth)acrylate having an acid group, the polymerizable composition can include water.

Examples and comparative examples were produced according to the blending ratio (% by weight) shown in Tables 2 to 4, and curability was evaluated under the humid condition.

Brevity codes in the tables are as follows. Bis-GMA: 2, 2-bis-4-(3-methacryloxy-2-hydroxypropyl) -phenylpropane

TEGDMA: Triethyleneglycol dimethacrylate

UDMA: Di-2-methacryloxyethyl-2, 2, 4-trimethylhexamethylene dicarbamate

GDMA: 2-Hydroxy-1, 3-dimethacryloxy propane

MDP: 10-methacryloyloxydecyldihydrogen phosphate

4MET: 4-Methacryloxyethyltrimellitic acid

CHP: Cumene hydroperoxide

t-BHP: tert-butyl hydroperoxide

BPO: Benzoyl peroxide

Di-LDAmCl: Dilauryl dimethyl ammonium chloride Cu(acac)2: Acetylacetone copper

SiO2 powder: Silica dioxide powder

Glass powder: Refer to Table 1 regarding glass powder blended as a filler

Aerosil: Fumed silica (the product name: R812, produced by Nippon Aerosil Corporation)

DW: Distilled water

IA: 6-tert-butyl-2,4-xylenol

NATU: N-acetylthiourea

NBTU: N-benzoylthiourea

v(acac)2: Vanadyl acetylacetonate

v(acac)3: Vanadium acetylacetonate

p-amine: p-tolyldiethanolamine

c-HexEtPTO: 1-cyclohexyl-5-ethylpyrimidinetrione TPO: 2,4,6-trimethylbenzoyl diphenylphosphine oxide

The blending ratio of the glass powder blended as a filler is shown in Table 1.

**<Table 1>**

| (% by weight) | Glass powder (Fluoroaluminosilicate glass powder) |
|---|---|
| Aluminum oxide | 21 |
| Anhydrous silicic acid | 44 |
| Calcium fluoride | 12 |
| Calcium phosphate | 14 |
| Strontium carbonate | 9 |

Powder (fluoroaluminosilicate glass powder) was produced by fully mixing raw materials shown in Table 1, holding the mixture in a high temperature electric furnace at 1200°C for 5 hours so as to fuse glass, cooling the fused glass, pulverizing the glass for 10 hours using a ball mill, and sieving the pulverized glass with a sieve of 200 meshes (ASTM). The powder was blended as a filler.

### [Evaluation for curability under the humid condition]

For evaluating curability under the humid condition, a compression test was carried out according to JIS/6609-1:2005 8.4 in each of examples and comparative examples. More specifically, the first component and the second component were kneaded at a weight ratio of 1:1. The mixture was filled in a metal mold having a diameter of 4mm and a height of 6mm, and cured for 1 hour under the humid condition (humidity of 100%). Then, the cured mixture was soaked in water at 37°C for 24 hours, and subjected to a compression test at a crosshead speed of 1 mm/min. In addition, usually, the compression test was carried out after 24 hours from the end of kneading. However, for simultaneously evaluating initial curability of the polymerizable composition according to the present invention, the compression test was carried out after 10 minutes from the end of kneading, too. In this compression test, the cement kneaded material was just cured under the humid condition, and was not subjected to a process for soaking it in water at 37°C.

It was confirmed clearly from Tables 2 to 4 that the polymerizable composition according to the present invention is excellent in curability under the humid condition.

**<Table 2>**

| Examples | | | 1 | 2 | 3 | 4 | 5 | 6 | 7 | 8 |
|---|---|---|---|---|---|---|---|---|---|---|
| First component | (Meth)acrylate monomer | Bis-GMA | 65.98 | | 27.65 | | | 20.65 | | |
| | | TEGDMA | 32.99 | 29.32 | 13.82 | 13.66 | 10.49 | | | |
| | | UDMA | | 58.65 | | 27.31 | 20.98 | | 15.98 | 13.98 |
| | | GDMA | | | | | | 10.32 | 7.99 | 6.99 |
| | (Meth)acrylate having an acid group | MDP | | 10 | | | 10 | 10 | 5 | 5 |
| | | 4MET | | | | | | | 12 | 12 |
| | Hydroperoxides | CHP | 1 | 2 | 05 | 1 | 05 | 1 | 1 | 1 |
| | Filler | SiO₂ powder | | | 55 | 55 | 55 | 55 | 55 | 55 |
| | | Aerosil | | | 3 | 3 | 3 | 3 | 3 | 3 |
| | Other additives | DW | | | | | | | | 3 |
| | | IA | 0.03 | 0.03 | 0.03 | 0.03 | 0.03 | 0.03 | 0.03 | 0.03 |
| | | Total | 100 | 100 | 100 | 100 | 100 | 100 | 100 | 100 |
| | | | | | | | | | | |
| Second component | (Meth)acrylate monomer | Bis-GMA | 65.74 | | 27.07 | | | 27.07 | | |
| | | TEGDMA | 32.87 | 32.87 | 13.54 | 13.64 | 13.54 | | | |
| | | UDMA | | 65.74 | | 27.27 | 27.07 | | 27.27 | 27.27 |
| | | GDMA | | | | | | 13.54 | 13.64 | 13.64 |
| | Thiourea derivative | NATU | 1 | | 1 | | 1 | 1 | | |
| | | NBTU | | 1 | | 1 | | | 1 | 1 |
| | Vanadium compound v(acac)₂ | | 0.06 | | 0.06 | 0.06 | 0.06 | 0.06 | 0.06 | 0.06 |
| | | v(acac)₃ | | 0.06 | | | | | | |
| | Filler | SiO₂ powder | | | 55 | 55 | | | | |
| | | Glass powder | | | | | 55 | 55 | 55 | 55 |
| | | Aerosil | | | 3 | 3 | 3 | 3 | 3 | 3 |
| | Other additives | TPO | 0.3 | 0.3 | 0.3 | | 0.3 | 0.3 | | |
| | | IA | 0.03 | 0.03 | 0.03 | 0.03 | 0.03 | 0.03 | 0.03 | 0.03 |
| | | Total | 100 | 100 | 100 | 100 | 100 | 100 | 100 | 100 |
| | | | | | | | | | | |
| Compression strength [Mpa] | | After 10 minutes | 177 | 187 | 199 | 208 | 189 | 207 | 203 | 200 |
| | | After 24 hours | 233 | 239 | 265 | 273 | 270 | 265 | 252 | 270 |

**<Table 3>**

| Comparative examples | | | 1 | 2 | 3 | 4 | 5 | 6 |
|---|---|---|---|---|---|---|---|---|
| First component | (Meth)acrylate monomer | Bis-GMA | | | | | 65.98 | |
| | | TEGDMA | 13.66 | 10.32 | 13.88 | 10.55 | 32.99 | 29.32 |
| | | UDMA | 27.31 | 20.65 | 27.76 | 21.09 | | 58.65 |
| | | GDMA | | | | | | |
| | (Meth)acrylate having an acid groud | MDP | | 10 | | 10 | | 10 |
| | Hydroperoxides | t-BHP | | | | | 1 | 2 |
| | Organic peroxide | BPO | 1 | 1 | | | | |
| | Organohalogen compound | Di-LDAmCI | | | 0.3 | 0.3 | | |
| | Copper ionic compound | Cu(acac)2 | | | 0.03 | 0.03 | | |
| | Filler | SiO₂ powder | 55 | 55 | 55 | 55 | | |
| | | Aerosil | 3 | 3 | 3 | 3 | | |
| | Other additives | IA | 0.03 | 0.03 | 0.03 | 0.03 | 0.03 | 0.03 |
| | | Total | 100 | 100 | 100 | 100 | 100 | 100 |
| | | | | | | | | |
| Second component | (Meth)acrylate monomer | Bis-GMA | | | | | 65.74 | |
| | | TEGDMA | 13.66 | 13.56 | 13.66 | 13.56 | 32.87 | 32.87 |
| | | UDMA | 27.31 | 27.11 | 27.31 | 27.11 | | 65.74 |
| | | GDMA | | | | | | |
| | Thiourea derivative | NATU | | | | | 1 | |
| | | NBTU | | | | | | 1 |
| | Vanadium compound vanadium compound | v(acac)₂ | | | | | 0.06 | |
| | | v(acac)₃ | | | | | | 0.06 |
| | Aromatic tert-amine | p-amine | 1 | 1 | | | | |
| | Pyrimidinetrione derivative | c-HexEtPT O | | | 1 | 1 | | |
| | Filler | SiO₂ powder | 55 | | 55 | | | |
| | | Glass powder | | 55 | | 55 | | |
| | | Aerosil | 3 | 3 | 3 | 3 | | |
| | Other additives | TPO | | 0.3 | | 0.3 | 0.3 | 0.3 |
| | | IA | 0.03 | 0.03 | 0.03 | 0.03 | 0.03 | 0.03 |
| | | Total | 100 | 100 | 100 | 100 | 100 | 100 |
| | | | | | | | | |
| Compression strength [Mpa] | | After 10 minutes | 140 | 136 | 146 | 150 | 120 | 135 |
| | | After 24 hours | 261 | 258 | 260 | 266 | 216 | 222 |

**<Table 4>**

| Comparative examples | | | 7 | 8 | 9 | 10 | 11 | 12 |
|---|---|---|---|---|---|---|---|---|
| First component | (Meth)acrylate monomer | Bis-GMA | 27.65 | | | 20.65 | | |
| | | TEGDMA | 13.82 | 13.66 | 10.49 | | | |
| | | UDMA | | 27.31 | 20.98 | | 15.98 | 13.98 |
| | | GDMA | | | | 10.32 | 7.99 | 6.99 |
| | (Meth)acrylate having an acid group | MDP | | | 10 | 10 | 5 | 5 |
| | | 4MET | | | | | 12 | 12 |
| | Hydroperoxides | t-BHP | 0.5 | 1 | 0.5 | 1 | 1 | 1 |
| | Organic peroxide | BPO | | | | | | |
| | Organohalogen comoound | Di-LDAmCI | | | | | | |
| | Copper ionic compound | Cu(acac)2 | | | | | | |
| | Filler | SiO₂ powder | 55 | 55 | 55 | 55 | 55 | 55 |
| | | Aerosil | 3 | 3 | 3 | 3 | 3 | 3 |
| | Other additives | DW | | | | | | 3 |
| | | IA | 0.03 | 0.03 | 0 03 | 0 03 | 0.03 | 0.03 |
| | | Total | 100 | 100 | 100 | 100 | 100 | 100 |
| | | | | | | | | |
| Second component | (Meth)acrylate monomer | Bis-GMA | 27.07 | | | 27.07 | | |
| | | TEGDMA | 13.54 | 13.64 | 13.54 | | | |
| | | UDMA | | 27.27 | 27 07 | | 27.27 | 27.27 |
| | | GDMA | | | | 13.54 | 13.64 | 13.64 |
| | Thiourea derivative | NATU | 1 | | 1 | 1 | | |
| | | NBTU | | 1 | | | 1 | 1 |
| | Vanadium compound | V(acac)₂ | 0.06 | 0.06 | 0.06 | 0.06 | 0.06 | 0 06 |
| | | V(acac)₃ | | | | | | |
| | Aromatic tert-amine | p-amine | | | | | | |
| | Pyrimidinetrione derivative | c-HexEtPTO | | | | | | |
| | Filler | SiO₂ powder | 55 | 55 | | | | |
| | | Glass powder | | | 55 | 55 | 55 | 55 |
| | | Aerosil | 3 | 3 | 3 | 3 | 3 | 3 |
| | Other additives | TPO | 0 3 | | 0.3 | 0.3 | | |
| | | IA | 0.03 | 0.03 | 0.03 | 0.03 | 0.03 | 0.03 |
| | | Total | 100 | 100 | 100 | 100 | 100 | 100 |
| | | | | | | | | |
| Compress ion strength [Mpa] | | After 10 minutes | 139 | 157 | 125 | 161 | 139 | 155 |
| | | After 24 hours | 259 | 268 | 267 | 255 | 238 | 260 |

## Claims

1. A polymerizable composition comprising:
a first component including cumene hydroperoxide as a peroxide in (meth)acrylate; and
a second component including a thiourea derivative as a reductant and a vanadium compound as a polymerization accelerator in (meth)acrylate.

2. The polymerizable composition as claimed in claim 1, wherein the composition further comprises:
a filler component in one or both of the first component and the second component.

3. The polymerizable composition as claimed in claim 1 or 2 wherein the thiourea derivative is one or more kinds selected from ethylenethiourea, diethylthiourea, tetramethylthiourea, N-acetylthiourea, N-benzoylthiourea, diphenylthiourea, and dicyclohexylthiourea.

4. The polymerizable composition as claimed in anyone of claims 1 to 3, wherein the vanadium compound is one or more kinds selected from vanadium acetylacetonate, vanadyl acetylacetonate, vanadyl stearate, vanadium naphthenate, and vanadium benzoyl acetonate.
